# NEW EUROPEAN PATENT SPECIFICATION

(11) **EP 0 227 795 B2**
(45) Date of publication and mention of the opposition decision: **24.07.2002**
(45) Mention of the grant of the patent: 15.12.1993
(21) Application number: 86904513.8
(22) Date of filing: 13.06.1986
(51) Int. Cl.: C12Q 1/68, G01N 33/53, C12N 15/00

(54) **METHOD FOR PERFORMING NUCLEIC ACID HYBRIDIZATION ASSAYS**
VERFAHREN ZUR DURCHFÜHRUNG VON HYBRIDISIERUNGSTESTS VON NUKLEINSÄUREN
PROCEDE D'EXECUTION D'ESSAIS D'HYBRIDATION D'ACIDES NUCLEIQUES

(30) Priority: 13.06.1985 US 744800
(43) Date of publication of application: 08.07.1987
(73) Proprietor: Amgen Inc.,, Thousand Oaks, California 91320-1799 (US); ABBOTT LABORATORIES, North Chicago, Illinois 60064 (US)
(72) Inventor: SNITMAN, David, L., Boulder, CO 80303 (US); STROUPE, Stephen, D., Libertyville, IL 60048 (US)
(74) Representative: Brown, John David
(86) International application number: US8601280
(87) International publication number: WO8607387

(56) References cited:
- EP-A- 0 139 489
- EP-A- 0 151 492
- EP-A- 0 155 854
- EP-A- 0 159 719
- EP-A- 0 192 168
- EP-A- 0 198 662
- GB-A- 2 169 403
- US-A- 4 256 834
- US-A- 4 358 535
- US-A- 4 455 380
- US-A- 4 478 914
- US-A- 4 486 539
- US-A- 4 556 643
- US-A- 4 563 417
- US-A- 4 581 333
- US-A- 4 582 789

## Description

### Background

The present invention relates in general to methods and kits for performing nucleic acid hybridization assays and in particular to methods and kits for immobilizing a target nucleic acid on a solid support by employing a labelled nucleotide probe, a nucleotide probe attached to a first complexing agent, and a second complexing agent attached to a support.

One characteristic property of nucleic acid, which forms the heritable material of all living organisms, is its ability to form sequence-specific hydrogen bonds with a nucleic acid having a complementary sequence of nucleotides. This ability of nucleic acids to form sequence-specific hydrogen bonds (i.e., to hybridize) with complementary strands of nucleic acid has been exploited in techniques generally called hybridization assays.

In a hybridization assay, a nucleic acid having a known sequence is used as a probe to search a sample for a "target" complementary sequence. Labelling of the hybrid formed by the probe and the target permits the detection and quantitation of complementary sequence in the sample.

Because all strains of a particular microorganism share a genetic component in the form of nucleic acids susceptible to diagnosis by means of a hybridization assay, such hybridization assays are valuable research and medical tools. Detection of specific target nucleic acids enables accurate diagnosis of bacterial, fungal and viral disease states in humans, animals and plants. Additionally, the ability to probe for a specific nucleotide sequence is of potential use in the identification and diagnosis of human genetic disorders.

One approach to labelling the probe for detecting a hybrid involves binding a radioisotope (e.g., ³²P or ¹²⁵I) to the probe.

Non-radioactive labelling systems are also available. A first type employs a label which may be directly and covalently attached to the probe, such as fluorescent or chemiluminescent molecules (e.g., fluorescein or acridinium). A second type has a portion which is covalently attached to the DNA probe and non-covalently attached to labelled macromolecules.

An example of the second type of non-radioactive labelling system involves a biotin molecule which is covalently attached to a DNA probe and which forms a complex with fluorescent- or chemiluminescent-"labelled" avidin (or avidin derivative such as streptavidin). Another example of the second type of non-radioactive labelling system is an antigen-"labelled" DNA probe which forms a complex with a fluorescent-or chemiluminescent-labelled antibody.

In the second type of labelling system, a probe is "labelled" with a reporter group to enable detection. A reporter is an agent which is used to associate a signal with a probe for indicating the presence or location of the probe. The signal itself, which is directly perceptible, may be generated by a separate or separable signal molecule. A label is properly a type of reporter which incorporates a signal.

Signal amplification may be achieved for biotin- or antigen-labelled DNA probes via the respective formation of a complex with avidin or with antibodies which may in turn be either covalently or non-covalently associated with an enzyme. [Leary, et al., Proc.Natl.Acad.Sci. (USA), 80: 4045-4049 (1983)]. This reporter group may then be incubated with the appropriate enzymatic substrate to generate a detectable signal which indicates the presence of target in the hybridization complex.

One approach to the attachment of labels to probes is described in Ward, European Patent Application No. 63,879. Ward discloses the preparation of probes having a biotin reporter molecule covalently attached to a purine or a pyrimidine ring. Selected biotinylated purines and pyrimidines are then directly incorporated within the phosphodiester backbone of nucleic acids of the probe by enzymatic means. In order to demonstrate that biotin-labelled native (double-stranded) DNA may be recognized by avidin, streptavidin or biotin-specific antibodies, Ward, et al. employ affinity chromatography. A complementary strand of DNA is synthesized on a single strand of DNA by a DNA polymerase from biotin- or iminobiotin-labelled purines or pyrimidines. The resulting, labelled, double-stranded DNA is selectively retained on an avidin-or a streptavidin-sepharose affinity column, as compared to non-labelled DNA. Ward, supra, at pages 24-26.

A biotin-labelled nucleic acid is employed in one approach to in situ hybridization in which biotin-labelled RNA is hybridized with denatured DNA in a chromosome squash. Polymethacrylate spheres are covalently attached to avidin which in turn binds to the biotin, thereby labelling portions of the DNA hybridized with the RNA. Manning, et al., Chromosoma (Berl.), 53: 107-117 (1975). In addition, avidin-coated, polymethacrylate spheres have been employed in affinity chromatography to isolate biotin-labelled strands of DNA carrying a particular gene. Manning, et al., Biochemistry, 16: 1364-1370 (1977).

In another approach to labelling for in situ hybridization, advantage is taken of the naturally-occurring bond between ribosomal protein and a pseudoribosomal gene in Drosophila. Antibodies are raised against the ribosomal protein and attached to polymethacrylate spheres which serve as labels for electron microscopy. Chooi, et al., Mol.Gen.Genet., 182: 245-251 (1981).

The formation of a complex between an antigenic substance being assayed and one or more antibodies is also the basis for another type of biological detection technique called an immunoassay. Antibodies are white blood cell-produced proteins which are capable of combining with an antigen in a reaction which is specific for that antigen. Both antigens and antibodies may be referred to as immunological agents. An antibody only combines with certain portions (antigenic determinants) of the surface of the antigen, so that the antibody is specific to the degree that the determinant with which it combines is not also found on other antigens. At least one member of the antigen/antibody complex may be coupled to a signal molecule which permits detection, quantitative analysis on separation of the antigen/antibody complex from uncomplexed labelled antigen or antibody and other constituents of the sample. Antibodies of any type may be employed in immunoassays including polyclonal antibodies, a mixture of antibodies directed to different antigenic determinants, and monoclonal antibodies, antibodies directed to a single antigenic determinant.

Both immunoassays and hybridization techniques are employed in two-site or "sandwich" assays. In sandwich assays a target substance having the ability to form hybrid or immune complexes at two different places on the target at one time is detected.

Typically, a sandwich immunoassay involves coupling a monoclonal antibody directed to a first antigenic determinant to a solid support and exposing the support-coupled antibody to a sample containing a substance bearing the first and a second antigenic determinant. This results in the removal of the antigenic substance from the sample by the formation of a primary antibody-antigen complex which is bound to the support. Subsequent exposure of this complex to a second, labelled monoclonal antibody directed toward a second antigenic determinant on the antigenic substance creates an antibody-antigen-antibody sandwich which may be separated from the sample solution and measured. [See, e.g., David, et al., U.S. Patent No.4,376,110.]

Sandwich hybridization assays include a two-step assay and a one-step assay. A two-step sandwich hybridization procedure involves the use of an immobilized target nucleic acid which is exposed in a first step to a first nucleic acid probe having a first portion complementary to the target and having a second portion which is not complementary to the target. In a second step, a second, labelled nucleic acid probe which is complementary to the second portion of the first probe is allowed to hybridize to the first probe, forming a "sandwich" with the first probe between the target and the second probe. Dunn, et al., Cell, 12: 23-36 (1977). The sandwich hybridization procedure is relatively easy to perform and is not seriously affected by protein or other biological contaminants. Ranki, et al., Gene, 21: 77-85 (1983). However, a two-step sandwich hybridization assay involves considerable delay associated with immobilization of the sample on a filter.

A one-step sandwich assay involves the use of a first nucleic acid probe immobilized on a filter. This first nucleic acid probe is complementary to a first portion of a target nucleic acid. In one step, the filter-bound first probe is exposed to a sample to be searched for the target nucleic acid sequence and to a second, labelled nucleic acid probe complementary to a second portion of the target nucleic acid, which portion is separate from (i.e., non-overlapping with) the portion of the target to which the first probe is complementary. Ranki, et al., U.S. Patent No. 4,486,539. This one-step technique eliminates the delay caused by immobilization of a sample on a filter; eliminates differences between the types of treatment required for binding ribonucleic acid (RNA) and deoxyribonucleic acid (DNA) to certain types of support inasmuch as the first probe may be selected to suit the support; and is even less sensitive to contaminating materials in the sample, e.g., mucus, than is a direct hybridization assay where the target is bound to the support. Ranki, et al., Curr.Top. Microbiol. Immunol., 104: 307-318 (1983). Nevertheless, leakage of the first probe from the support during hybridization occurs frequently and drastically diminishes the sensitivity of the assay.

Although both immunoassays and hybridization diagnostics are more rapid than conventional tests which require viable organisms and two to three days' culture, the antigens produced in a particular disease may vary from patient to patient and from one strain of a bacterium to another or from one strain of a virus to another, so that immunological diagnosis may be difficult. On the other hand, all strains of a bacterium or of a virus share a genetic component in the form of nucleic acids susceptible to diagnosis through the use of a nucleic acid probe.

Nevertheless, it is neither easy nor convenient to attach a single-stranded nucleic acid probe directly to a solid support for use in a sandwich hybridization assay. For example, the attachment of a nucleic acid to a nitrocellulose sheet involves fixing the nucleic acid by contact with the sheet for 12-15 hours and baking the nucleic acid onto the sheet for two hours. See, e.g., Thomas, Proc. Natl. Acad. Sci.(USA), 77: 5201 (1980). Such preparation of a DNA-coated nitrocellulose sheet may easily consume as much as a full working day, a factor which limits the clinical usefulness of nucleic acid hybridization.

Furthermore, because the nucleic acid probe is sequence-specific for a particular target molecule, the procedure for attaching the probe to the support must be performed for each target molecule to be detected. Thus, in order to detect a number of different DNA sequences, a diagnostic laboratory must prepare an equal number of types of supports.

In addition, it generally takes longer to hybridize complementary strands of nucleic acid than it does, for example, to form an immunological complex between an antigen and an antibody Hybridization itself is much more quickly accomplished in solution than it is where one of the complementary sequences is attached to a solid support.

Affinity chromatographic techniques may be employed to isolate and purify nucleic acids [see, e.g., Inouye. et al., J.Biol.Chem., 23: 8125-8129 (1973)) or tRNA [Miller, et al., Biochim.Biophys.Acta, 366: 188-198 (1974)] or tRNA cistrons [Salomon, et al., Biochemistry, 14: 4046-4050 (1975)]. However, these techniques rely upon the difficult step of forming antibodies to specific bases in a nucleic acid (Inouye, et al., supra: Salomon, et al., supra) or upon the use of a derivatized, naturally-occurring ribonucleic acid (tRNA) (Miller, et al., supra) and are thus not readily applied in general to hybridization assays.

Thus, there exists a continuing interest and need in the art for easy, convenient and rapid nucleic acid hybridization "sandwich" assays capable of accurately detecting target molecules in a sample.

### Brief Summary

According to the present invention as claimed for BE, CH, DE, FR, GB, IT, LI LU, NL and SE, there is provided a method for the isolation of a selected target nucleic acid sequence from a solution, said method involving the formation of a sandwich complex of:
(a) a first single-stranded nucleic acid probe hybridized to a complementary first portion of the target, said probe first being attached to a first complexing agent for immobilizing said probe to a solid support bearing a second complexing agent capable of stably binding to the first complexing agent; (b) the target nucleic acid sequence ; and (c) a second single-stranded nucleic acid probe hybridized to a complimentary second portion of the target, said second probe being attached to a reporter group; characterised in that: said sandwich second complex is formed in solution prior to immobilization of the first probe to the solid support; in that said first and second complexing agents selected from the group of specific binding pairs consisting of an antigen and an antibody, and a lectin and a carbohydrate and in that said first complexing agent is attached to said first single-stranded nucleic acid probe at the 5' end thereof, and wherein the specific binding pairs do not consist of sulphonated DNA as the antigen and anti-sulphone monoclonal antibodies as the antibody.

According to the present invention as claimed for at, AT, there is provided a method for the isolation of a selected target nucleic acid sequence from a solution, said method involving the formation of a sandwich complex of:
(a) a first single-stranded nucleic acid probe hybridized to a complementary first portion of the target, said first probe being attached to a first complexing agent for immobilizing said probe to a solid support bearing a second complexing agent capable of stably binding to the first complexing agent; (b) the target nucleic acid sequence ; and (c) a second single-stranded nucleic acid probe hybridized to a complimentary second portion of the target, said second probe being attached to a reporter group; characterised in that: said sandwich second complex is formed in solution prior to immobilization of the first probe to the solid support; in that said first and second complexing agents selected from the group of specific binding pairs consisting of an antigen and an antibody, and a lectin and a calbohydrate and in that said first complexing agent is attached to said first single-stranded nucleic acid probe at the 5' end thereof.

A method according to the present invention for the isolation and quantitative detection of a selected target nucleic acid sequence from solution involves hybridizing the target nucleic acid sequence in solution to a first single-stranded nucleic acid probe which has a sequence complementary to a selected portion of the target sequence and which is therefore capable of hybridizing therewith. The first probe sequence is covalently attached to a first complexing agent. A second single-stranded nucleic acid probe, which has a sequence complementary to a different selected portion of the target sequence than that which is complementary to the first probe, hybridizes to the target. A detectable reporter group is attached to the second probe sequence.

Following solution hybridization, the method according to the present invention further involves immobilizing the hybrid sequence by adding to the hybridization solution a second complexing agent bound to a solid support capable of binding to the first complexing agent on the first probe. There is thus obtained a sandwich comprising the second complexing agent-support, complexed with the first complexing agent-first probe hybridized to the target, in turn hybridized to the second probe. An assay is then performed to detect and quantitate the bound reporter.

The present invention is used for performing a hybridization assay on a sample containing a selected target nucleic acid sequence from solution. A first probe has a nucleic acid sequence complementary to a first portion of the target nucleic acid sequence and is attached to a first complexing agent. A second single-stranded nucleic acid probe associated with the first nucleic acid probe has a nucleic acid sequence complementary to a second portion of the target sequence and is associated with the first probe. A reporter group is attached to the second nucleic acid probe. A solid support, also associated with the first nucleic acid probe, is attached to a second complexing agent which has a first complexing agent-binding portion.

Another method according to the present invention increases the capture efficiency associated with immobilizing a target nucleic acid sequence on a solid support. This method involves exposing the target nucleic acid sequence to at least two first probes, each having a nucleic acid sequence complementary to a different portion of the target nucleic acid sequence and each having a support-binding portion. In solution, the target nucleic acid sequence is hybridized to at least one of the first probes. The support-binding portion of the at least one of the first probes attaches to a first probe-binding portion on a solid support.

The present invention is useful for performing a hybridization assay on a sample containing a target nucleic acid sequence. At least two first probes are needed, each of which has a nucleic acid sequence complementary to a different portion of the target nucleic acid sequence. A second probe is associated with the first probes. The second probe has a sequence which is complementary to a portion of the target nucleic acid sequence that is separate from any portion complementary to any first probe. The second probe is also attached to a reporter group. A solid support is also associated with the first probes and has a first probe-binding portion.

Other aspects and advantages of the present invention will become apparent to those skilled in the art upon consideration of the following detailed description.

### Detailed Description

In a preferred embodiment of the method according to the present invention, a target nucleic acid sequence in solution may be detected or quantified by measuring the amount of a signal associated with an immobilized "sandwich" hybrid by conventional methods. The method is also useful in separating a hybridized target sequence from a solution where detection is not required. The method according to the present invention may be employed where the target oligonucleotide sequence is a deoxyribonucleic or ribonucleic acid sequence. In either case, depending on preference for a DNA-DNA, RNA-RNA, or DNA-RNA hybridization between the first probe, labelled second probe, and target, the probe sequences may be deoxyribonucleic or ribonucleic acid sequences.

The method is desirably employed on a double-stranded target sequence when the double-stranded sequence is denatured prior to use in the hybridization and is also useful for detection of single-stranded target sequences. The target sequence employed in this method may be of any length but preferably greater than about 20 residues in length.

The first probe sequence itself may be any nucleic acid sequence capable of covalently binding to the selected first complexing agent and having at least a portion designed to complement and to stably hybridize with a portion of the target sequence. The first complexing agent, which is covalently attached to the first probe, may be an antigen, such as fluorescein or an antibody, such as anti-fluorescein; or may be biotin or avidin; or may be a lectin, such as concanavalin A or a carbohydrate having, for example, α-glucosyl residues or α-mannosyl residues specific for concanavalin A.

A lectin is a protein which has combining groups that react with specific carbohydrate components of another molecule to form a complex in a fashion similar to the interaction of an antibody with an antigen. Biotin, a vitamin, is an imidazole derivative which combines with avidin, a protein found in eggwhite, to form a biotin-avidin complex. Thus antigens and the antibodies which bind to them, lectins and the carbohydrate components to which they bind, and biotin and avidin, all may be distinguished as complexing agents, forming non-covalent bonds, from nucleic acids which are sequence-specific hybridizing agents, forming hydrogen bonds.

Several techniques may be employed to generate single-stranded polynucleotide probes for use in hybrization. A probe sequence complementary to a desired "target" sequence may be obtained: as a messenger RNA sequence corresponding to a target sequence; or complementary DNA obtained from reverse transcription of messenger RNA by the enzyme reverse transcriptase; or as genomic DNA obtained from the target genome by endonuclease digestion.

A probe sequence may be "amplified" by insertion into a DNA plasmid, such as pBR322, which will replicate in a bacterial host cell. Plasmid DNA is double-stranded and may be labelled by well-known nick translation procedures.

Alternatively, a probe sequence may be amplified by inserting the desired sequence into a single-stranded virus, such as the bacteriophage M13. The virus containing the probe sequence thereafter infects the bacterial culture and multiplies, making billions of copies of the probe sequence attached to viral DNA. The viral clone DNA may be isolated as either single-stranded or double-stranded DNA. Double-stranded viral DNA may be labelled by nick translation. Single-stranded viral DNA may be rendered detectable through use of primed synthesis of complementary strand DNA using labelled nucleotides according to the procedures of Hu, et al., Gene, 17: 271-277 (1982). See Ranki, et al., Gene, 21: 77-85 (1983), which relates to M13 and pBR322 amplification systems for generating single-stranded probes for use in sandwich hybridization assays.

Like the first probe, the second probe may have any nucleic acid sequence which is different than that of the first probe and which is designed to complement and hybridize with the target at a portion of the target separate from (i.e., not overlapping) the portion to which the first probe hybridizes.

A reporter group may be covalently attached to the second probe. The reporter group may be a radioisotopic label, such as ¹²⁵I, ³²P, or the like. Alternatively, chelating moieties, such as ethylene diamine tetraacetic acid (EDTA) or diethyltriamino pentacetic acid (DTPA), may be employed to attach heavy metal labels to the probe. Appropriate heavy metal labels include ⁵⁷Co, ⁶³Ni, ¹¹¹In, ⁹⁹Tc, ⁵⁵Fe, ⁵¹Cr, and the like. Non-isotopic labels, e.g., fluorescent compounds and chemiluminescent compounds, may also be employed in the method according to the present invention. Non-radioactive reporter groups which may be attached to the second probe include the enzyme alkaline phosphatase linked, for example, by biotin or avidin, to the second probe. Incubation in a solution of a methylumbelliferone phosphate substrate results in fluorescence produced by the action of the enzyme on the substrate.

Any solid support to which a complexing agent may be bound is useful in this method, including both porous and non-porous, polymeric and non-polymeric supports. Examples of a solid support suitable for use in the method include silicates in general and glass, silica gel, and controlled pore glass in particular; cellulose and nitrocellulose paper; polystyrene; latex and rubbers; and fluorocarbon resins, such as Teflon® and the like.

The second complexing agent bound to the support may be any agent which forms a complex with the first complexing agent on the first probe. For example, the second complexing agent may be an antibody (e.g., IgG, IgM, or IgA), including a monoclonal antibody such as anti-fluorescein antibody where the antigen on the first probe is fluorescein.

As is clear to one skilled in the art, the present invention provides several advantages over the conventional attachment of a first nucleic acid probe to a solid support. Because a single combination of first and second immunological agents may be used with a wide variety of probe and target sequences, the present invention eliminates the need for a laboratory to prepare a support specific to each sequence to be detected. Furthermore, to the extent that hybridization according to the present invention occurs between complementary strands in solution rather than between one in solution and another on a solid support, the hybridization procedure proceeds more quickly. Furthermore, complex formation is much more rapid than hybridization so that the use of complex formation rather than hybridization to attach the target to a support reduces assay time even further. Also, attachment of antibodies, antigens, lectins, carbohydrates, biotin or avidin to a solid support does not require as many steps and is not as time-consuming as is the attachment of a sequence of nucleic acid to a solid support. Cf., e.g., Thomas, Proc. Natl. Acad. Sci. (USA), 77: 5201-5205 (1980).

Thus, the present invention provides the means for accomplishing hybridization diagnostic tests much more easily, rapidly, and conveniently.

The following examples illustrate the practice of the method of the present invention. Specifically demonstrated are hybridization assays employing the two-probe system to detect and quantitate the amount of desired target sequence in a solution.

For use in the solution hybridization procedures of the following examples, a single-stranded phage containing either the (+) plus (coding) strand or the (-) minus (anticoding) strand of the Herpes Simplex Virus Type I (HSV-I) glycoprotein D (gD) gene was employed as the target sequence. A portion of the double-stranded gene sequence is set out in Table I below, the bottom strand being the anticoding strand. This sequence has been published in Watson, et al., Science, 218: 381-384 (1982). Portions of the plus and minus strands have been employed as probes according to the present invention. These single-stranded probe sequences have been designated on Table I by a lettered line drawn above the coding strand of the gene, or by a lettered and numbered line drawn below the anticoding strand of the gene.

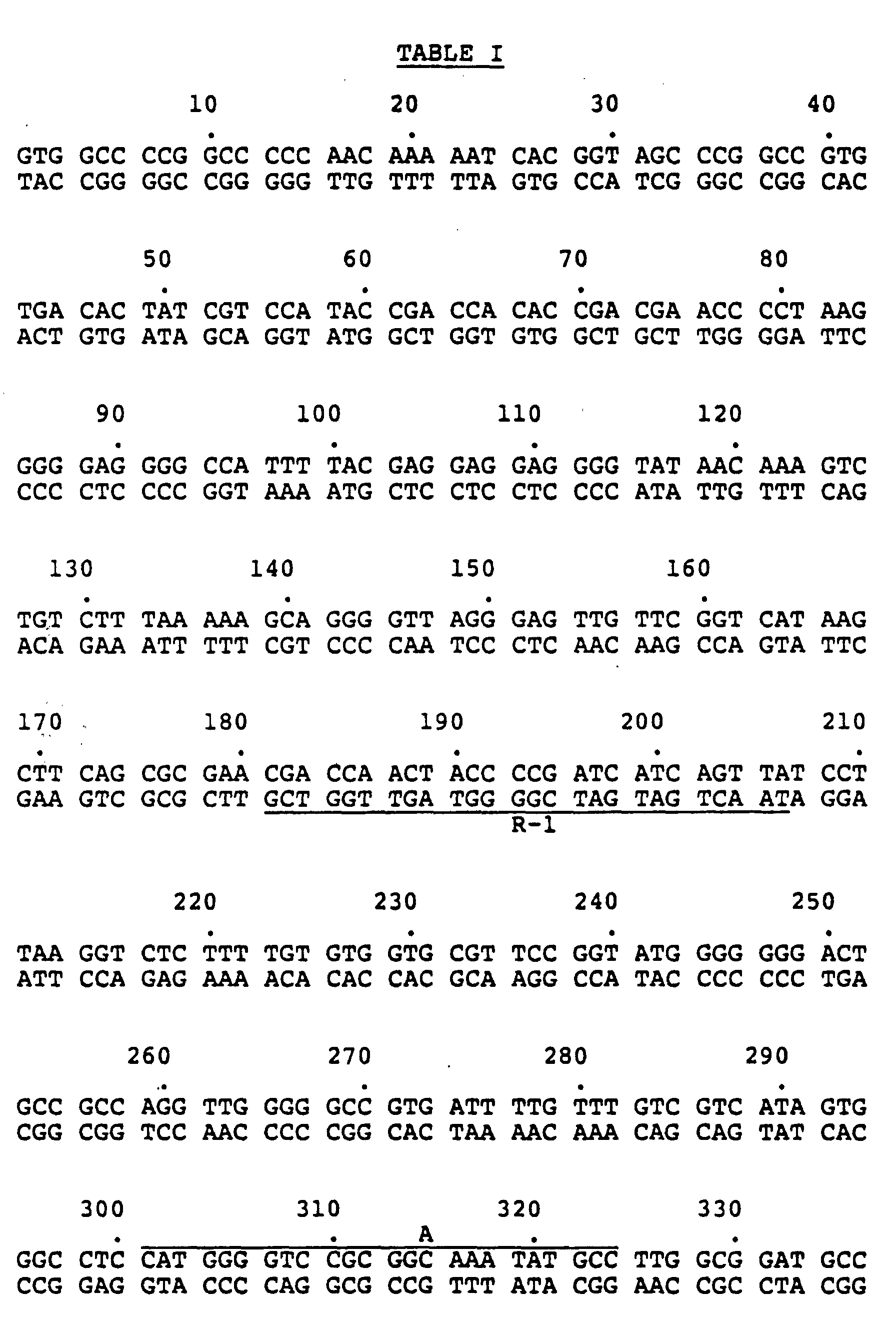

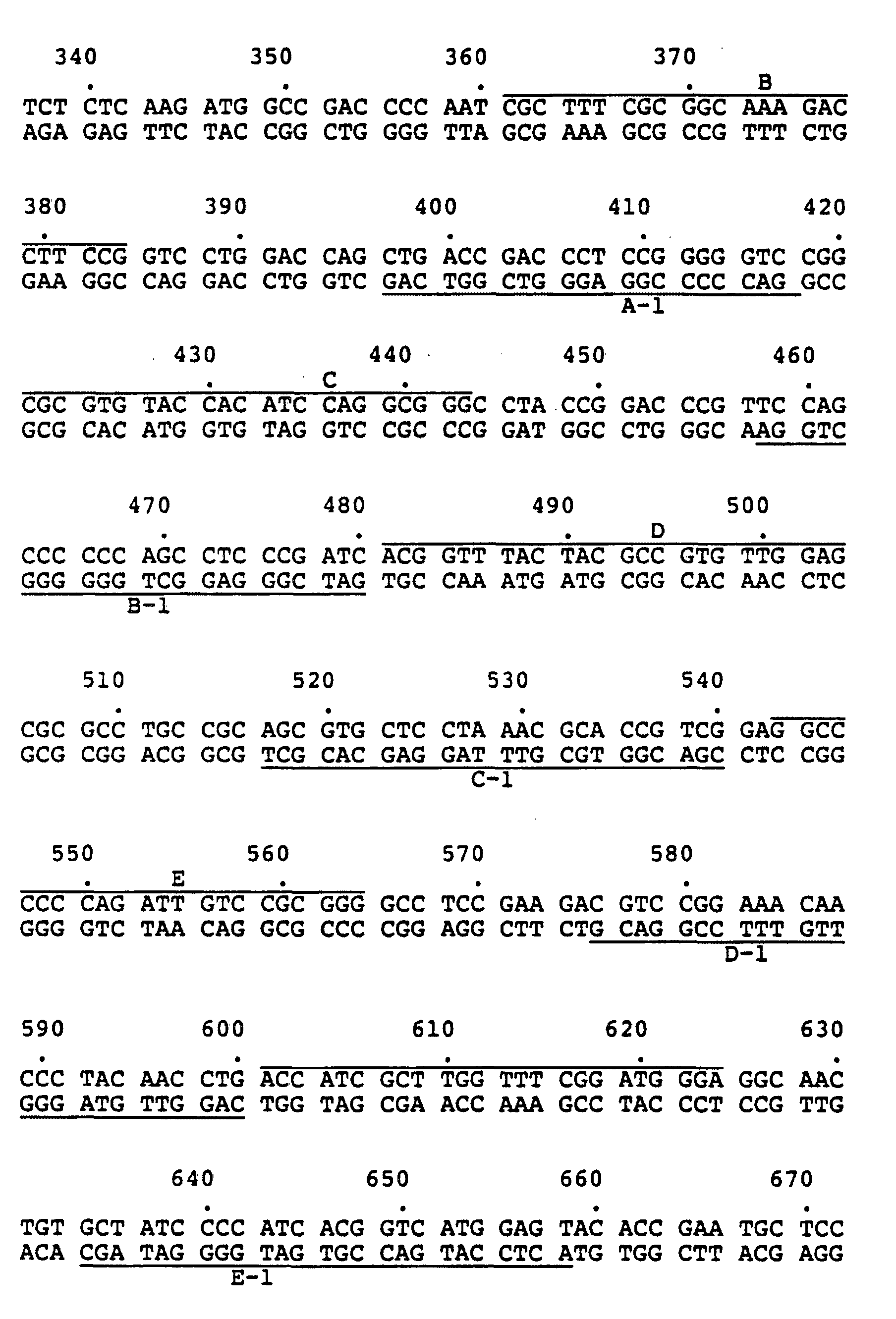

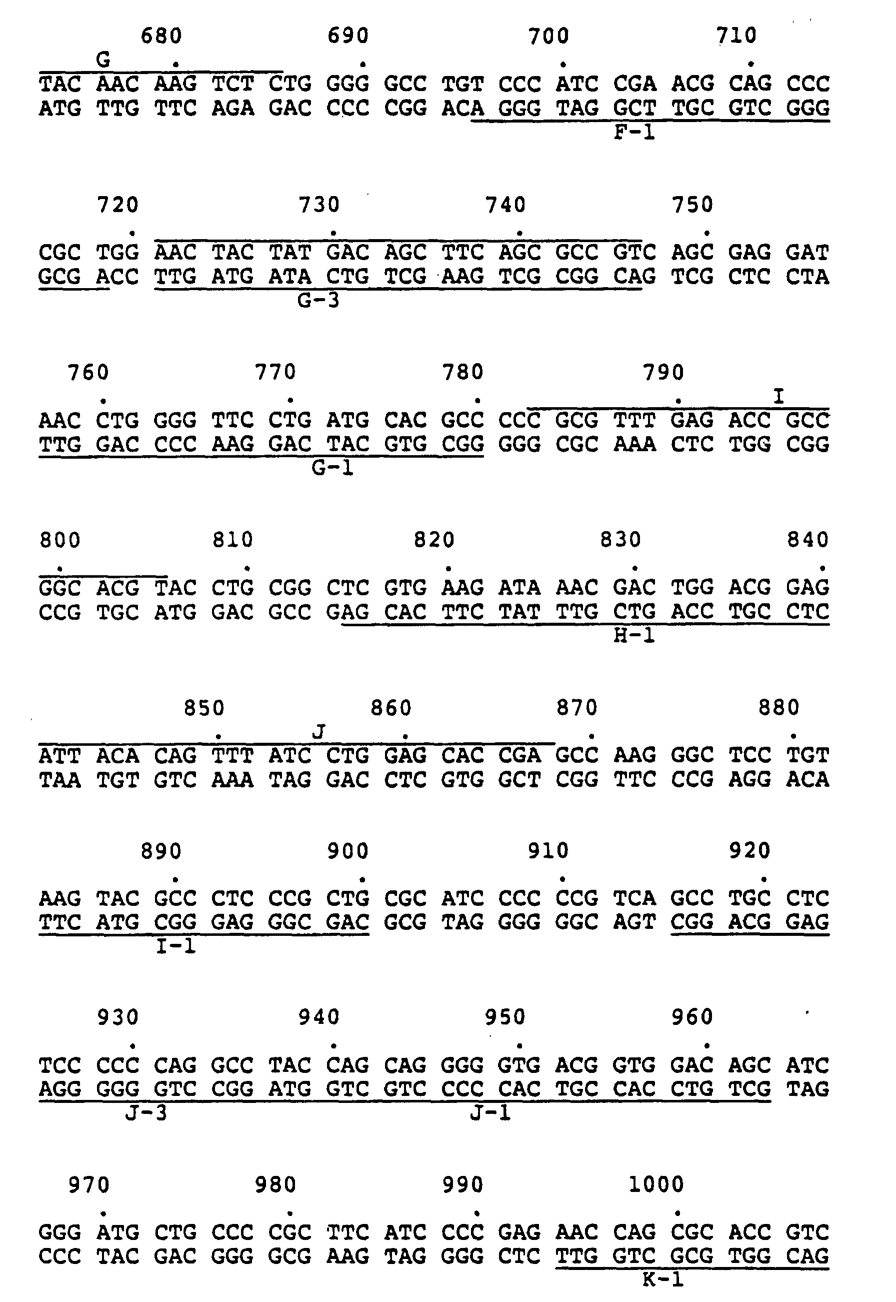

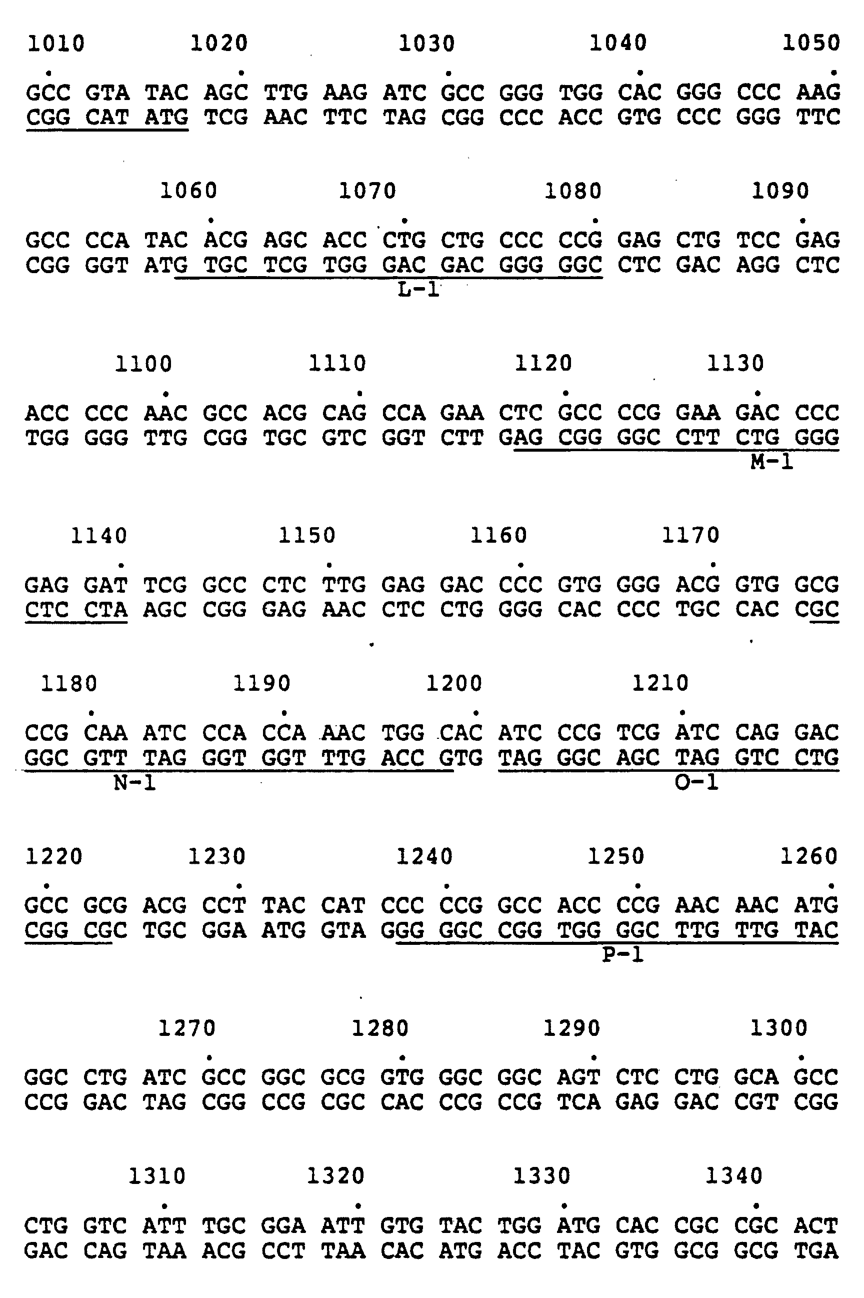

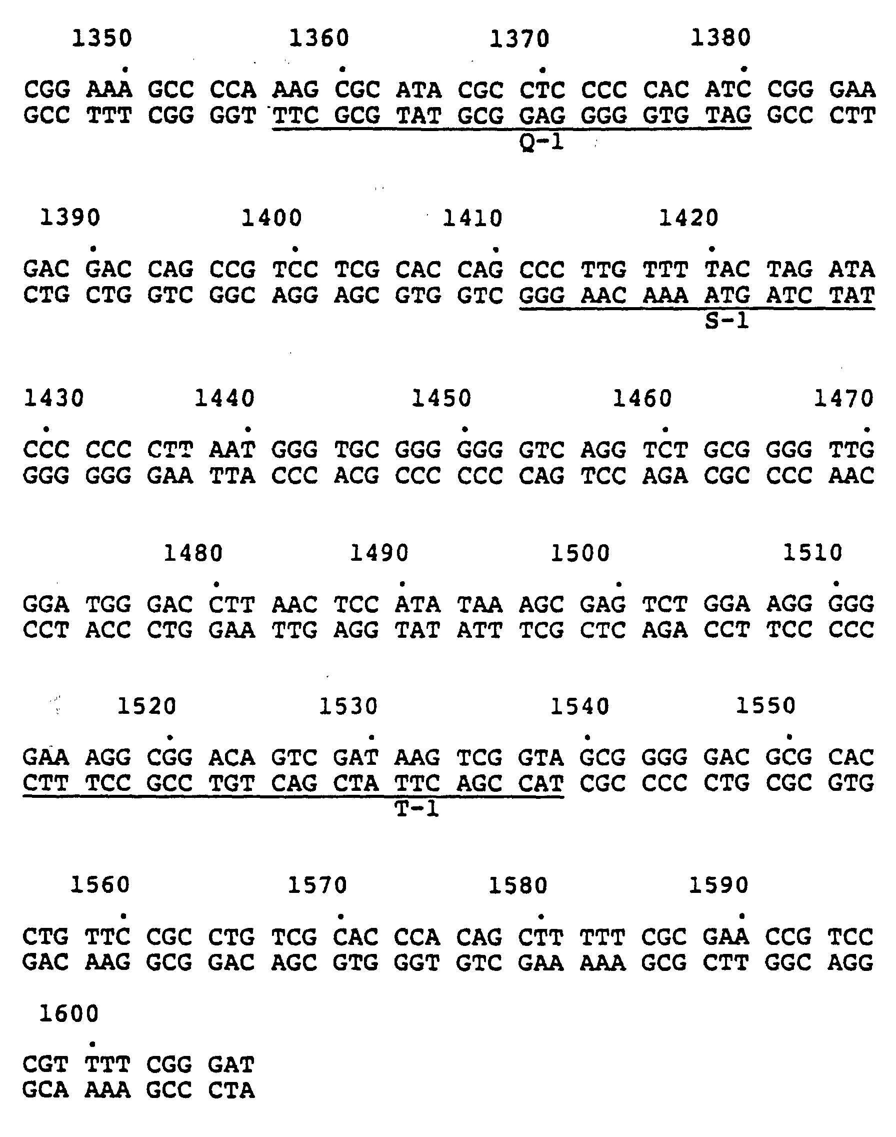

Three different targets are used in the examples. A first single-stranded phage target, phage 2 (Φ2), contains 1,360 bases of the HSV-I D (gD) gene (i.e., bases 167 through 1,526, initiation codon nucleotide number 241 cloned into a plasmid, M13mp18. The minus strand sequence of gD in Φ2 is employed as a target complementary to the (+) plus strand probes identified above. A second single-stranded phage target, NPE #1, contains the entire 2.9 kilobases of the HSV-I gD sequence and is cloned into M13mp18. The (+) plus strand sequence of gD in NPE #1 is cloned to provide a target complementary to the (-) minus probes identified above. Lastly, a double-stranded plasmid target, BamHI-J, is a BamHI restriction fragment of HSV-I which contains the entire 2.9 kilobases of the HSV-IgD sequence, along with 3.3 kilobases of surrounding HSV-I sequences. BamHI-J was cloned into the plasmid pBR322 and this plasmid was used as a double-stranded target for mimicking hybridization to HSV-I virus. See Roizman, et al., Curr. Top. Microbiol. Immunol., 104: 273 (1983).

The following examples describe a series of experiments demonstrating various aspects of the present invention.

Example 1 shows the ability of an antibody-coated support to capture a hybridization sandwich comprising two probes bound to a target. Example 2 illustrates the increase in capture efficiency obtained through the use of multiple antigen labelled probes. Example 3 demonstrates the effects of target concentration on the efficiency and sensitivity of the hybridization assay according to the present invention.

### Example 1

The ability of an antibody-coated solid support to capture a hybridization sandwich formed by two probes and a target was tested. An oligonucleotide first probe was labelled with an antigen at its 5' end. A second probe was an oligonucleotide carrying a reporter group. A portion of the target was complementary to each probe.

Specifically, the first probe was oligonucleotide G as described above. Such 5' labelling of oligonucleotide G may be accomplished with fluorescein.

Oligonucleotide G was 5' fluorescein labelled by reacting a 5' amine functionalized oligonucleotide G with fluorescein isothiocyanate. The 5' amine functionalized oligonucleotide G was formed by reacting oligonucleotide G bound by its 3' end to a solid support with a phosphoramidite having the general formula [(CH₃)₂CH]₂NP(OCH₃)O(CH₂)₈NH(DMT) wherein DMT is a dimethoxytrityl group.

In the synthesis of this phosphoramidite, about 8ml of diazomethane-ether solution were added to 159.2 mg (1 mmole) of ω-aminocaprylic acid (available from Aldrich Chemical, Milwaukee, Wisconsin) in 10 ml of methanol. The methanol was evaporated to yield 174.9mg of ω-aminocaprylic acid methyl ester. Next, 173mg (1 mmole) of the ω-aminocaprylic acid methyl ester, 1 mmole of dimethoxytrityl chloride, and 1 mmole of diisopropylethyl amine were added to 5 ml of anhydrous tetrahydrofuran under an argon atmosphere at 0°C. This mixture was warmed to 25°C and stirred for 1 hour. The solvent was evaporated and the crude product was diluted with 50 ml of ethyl acetate and washed successively with two portions of water, saturated bicarbonate, and brine. The product was dried over anhydrous magnesium sulfate and evaporated to yield 460 mg of a dimethoxytrityl derivative of the ω-aminocaprylic acid methyl ester (ACAM-DMT).

To 0.17mmoles of ACAM-DMT in 1ml of anhydrous tetrahydrofuran under an argon atmosphere at -78°C was added 1.24 ml of 1 molar lithium aluminum hydride in tetrahydrofuran. This reaction mixture was stirred for 5 minutes at -78°C and was then stirred for 30 minutes at 25°C before being diluted with 10 ml of 5% H₂O in tetrahydrofuran, 200 ml of ether, 3 g of cellite, and 0.5 g of anhydrous magnesium sulfate. The resulting mixture was stirred for 30 minutes and filtered to yield an alcohol having the general formula HO(CH₂)₈NH-DMT.

To 0.72 mmoles of HO(CH₂)₈NH-DMT in 10ml of anhydrous dichloromethane was added 0.76 mmoles of diisopropyl ethyl amine and 0.76 mmoles of chloro-N.N'-diisopropylaminomethoxy phosphene (as available from American Bionuclear, Emeryville, California). This mixture was stirred for 40 minutes at 25°C, and then diluted with 50 ml of ethyl acetate and washed with four portions of brine. The product of this reaction was the phosphoramidite used for labelling oligonucleotide G above.

The second probe was oligonucleotide A, which had been labelled with ³²P according to the procedure of Maniatis. et al., Cell. 15: 687 (1978). The specific activity of the probe on the date of use was 3.2x10⁶ cpm pmole.

Oligonucleotide G without a 5' fluorescein label was used as a first probe control. A second control probe, having the sequence 5'CATGATCTTGCGGTCGGATTCTTC3', which does not complement any of the target sequence, was also ³²P-labelled and had a specific activity on the date of use of 3.2x10⁶ cpm/picomole.

The target used was single-stranded Φ2. Single-stranded Φ2 is complementary to the first and second probes and to the first probe control, but not to the second control probe.

As a support, one-quarter-inch polystyrene beads of the sort available from Pierce Chemical, Rockland, Illinois, were coated with fluorescein antibody (anti-fluorescein), Anti-fluorescein production was induced in rabbits. The anti-fluorescein was purified by ammonium sulfate precipitation, followed by DEAE cellulose chromatography. In solution, the anti-fluorescein had an affinity of approximately 10¹² and quenched the fluorescence of fluorescein by about 99%.

To prepare an anti-fluorescein-coated bead, the bead is cleaned by ultrasonication for 15 seconds in 10mM NaHCO₃ buffer at pH 8. After ultrasonication, the beads are washed in deionized water until all fines are removed. Approximately 200 beads are covered by 40 ml of 10 mM NaHCO₃. Next, 7 ml of purified anti-fluorescein at a concentration of 0.57 mg/ml is added. The beads are incubated for approximately 65 hours at room temperature. After incubation, the beads are washed with deionized water and air-dried on a suction filter.

Each of the anti-fluorescein-coated beads is capable of binding approximately 1 pmole of fluorescein, as demonstrated by incubation of single beads with 1.5ml of 1 nM fluorescein in TDX buffer (0.1 M NaPO₄, pH 7.5; 0.1% NaN₃; 0.1% bovine gammaglobulin). During 20 hours of incubation at 25°C, 97% of the fluorescence was removed from solution. After washing the beads three times in 5ml of deionized water and blotting the beads dry after each wash, the beads were incubated in 0.1 M NaOH for 10 minutes, in which 60% of the originally applied amount of fluorescein was released into solution. Thus, each bead has approximately 0.9 pmole of fluorescein binding capacity.
(1) A series of capture experiments employing 5'-fluorescein-labelled oligonucleotides, 5'-biotin-labelled oligonucleotides (both 3'-³²P end-labelled), and kinased ³²P-labelled oligonucleotides and polystyrene beads coated with anti-fluorescein were run under the following conditions.
With 200µg/ml denatured sheared salmon sperm DNA (Sigma Chemical Company, St. Louis, Missouri) containing 1 picomole of one of the ³²P-labelled oligonucleotides, 100µl of TDX buffer (0.1M sodium phosphate, pH 7.5; 0.1% NaN₃; and 0.01% bovine gamma globulin, Sigma Chemical Company, St. Louis, Missouri) was mixed. An anti-fluorescein-coated polystyrene bead was added to this solution. After incubating this system for 18 hours at 25 °C, the bead was removed and washed for 5 minutes in 1 ml of TDX buffer at 25 ° C. The bead was then counted in a scintillation counter.
The stability of the antibody complex on the bead was tested by washing the bead for 5 minutes at increasing temperatures. The capture efficiency and stability of a series of such beads is shown in Table II.

**TABLE II**

| Percent cpm Capture | | | |
|---|---|---|---|
| Temperature | Complexes | | |
| | 5' fluorescein-labelledcomplex | 5' biotin-labelled complex | 5' ³²P-labelled complex |
| 25 | 63 | 4 | 3 |
| 35 | 61 | 1 | 0 |
| 45 | 56 | 0 | 0 |
| 55 | 51 | 0 | 0 |
| 65 | 42 | 0 | 0 |
| 75 | 35 | 0 | 0 |
| 85 | 20 | 0 | 0 |
| 95 | 0 | 0 | 0 |

As illustrated by Table II, these beads have a high capture efficiency and stability of the sort which is useful in a hybridization capture system. Because little or no biotin or ³²P-labelled oligonucleotide binds to these beads, indicating little non-specific binding to the beads, the background in such a system is very low.
(2) In order to more precisely determine the rate of capture of a fluorescein-labelled oligonucleotide by a fluorescein antibody-coated bead, each of a series of beads was incubated for a different amount of time with 1 picomole of 5'-fluorescein-labelled oligonucleotide A which had been 3' end-labelled with ³²P. The percent of capture was determined for each bead and the results are shown below in Table III.

**TABLE III**

| Time | Percent Oligonucleotide Capture |
|---|---|
| 0 | 0 |
| 15 minutes | 20 |
| 30 minutes | 45 |
| 1 hour | 48 |
| 2 hours | 75 |
| 3 hours | 91 |
| 4 hours | 90 |
| 5 hours | 88 |
| 6 hours | 86 |
| 7 hours | 85 |
| 8 hours | 82 |
| • | |
| • | |
| • | |
| 20 hours | 68 |

As illustrated in Table III, 90% of the 5' fluorescein-labelled oligonucleotide is captured by the bead in 2 to 3 hours. The slow decline in the amount of radiolabel over time on the bead most likely represents a small amount of leakage of the antibody from the bead.
(3) Experiment 1. The capture-efficiency of the anti-fluorescein-coated beads being established, 1 picomole of the first probe (5'-fluorescein-labelled oligonucleotide G), 1 picomole of the second probe (³²P-labelled oligonucleotide A), specific activity on date of use 3.2x10⁶ cpm/picomole), and 1 picomole of the target (Φ2 SS, complementary to both the first and second probes) were diluted to 50µl with 5 X SSPE diluted from 20 X SSPE (3.6M NaCI; 0.23 M NaH₂PO₄, pH 7.5; and 20 mM EDTA). This hybridization solution was incubated for 3 hours at 50°C. This hybridization solution was diluted with 100µl of TDX buffer and one anti-fluorescein-coated bead was added. After incubation for 3 hours at 25 ° C, the bead was washed with 1 ml of TDX buffer for 5 minutes at 37 °C and was rewashed with 1ml of TDX buffer for 5 minutes at 37C before counting in a scintillation counter.

Control Experiments. Three control experiments were run according to the same protocol but with the following modifications. In a first control experiment (Control 1), 5' fluorescein-labelled oligonucleotide G, as a first probe, and 5' ³²P-labelled oligonucleotide A, as a second probe, were incubated with the anti-fluorescein-coated bead in the absence of any target. A second control experiment (Control 2) involved the use of 1 picomole of unlabelled oligonucleotide G as a first probe for the fluorescein-labelled oligonucleotide G of experiment 1. Finally, a third control experiment (Control 3) was performed with 1 picomole of 5'-fluorescein-labelled oligonucleotide G, as a first probe, 1 picomole of a ³²P-labelled oligonucleotide designated 32-B₂ (the sequence of which is not complementary to Φ2 SS), as a second probe, and 1 picomole of Φ2 SS as a target.

The results of these experiments are summarized in Table IV.

**TABLE IV**

| Experiment | % ³²P Oligonucleotide Bound to the Bead |
|---|---|
| Experiment 1 | 4.2 |
| Control 1 | 0.002 |
| Control 2 | 0.07 |
| Control 3 | 0.22 |

A comparison of Experiment 1 and Control 1 indicates that the hybrid comprising fluorescein-labelled oligonucleotide G, Φ2 SS, and ³²P-labelled oligonucleotide A may be selectively captured by an anti-fluorescein-coated solid support. Controls 2 and 3 demonstrate that in the absence of the correct antigen-labelled first probe or in the absence of the correct target complementary second probe, a hybrid is not effectively generated or captured.

### Example 2

In an attempt to increase the capture efficiency of the hybridization assay according to the present invention, several fluorescein-labelled oligonucleotide probes were simultaneously introduced into the hybridization solution. Four experiments were run under identical reaction conditions.

A total of 250 femtomoles of fluorescein-labelled oligonucleotide was used in each experiment. In Experiment 1, 250 femtomoles of a single fluorescein-labelled oligonucleotide were used. The hybridization solution of Experiment 2 contained 125 femtomoles of each of two different fluorescein-labelled oligonucleotides, while Experiment 3 involved 83 femtomoles of each of three different fluorescein-labelled oligonucleotides in the hybridization solution. In Experiment 4, the hybridization solution contained 28 femtomoles of each of nine different fluorescein-labelled oligonucleotides.

Specifically, in Experiment 1, a 5XSSPE solution of 250 femtomoles of 5' fluorescein-labelled oligonucleotide B, 25 femtomoles of a target Φ2 SS and 100 femtomoles of ³²P-labelled oligonucleotide A was boiled for 5 minutes to denature any double-stranded secondary structure which might be present and incubated at 50°C for 3 hours. The hybridization solution was diluted with 50µl of 5XSSPE before adding one anti-fluorescein-coated bead. The bead was incubated in this solution for 4 hours at 25°C and was washed in 1 ml of 5 X SSPE for 5 minutes at 25 ° C before counting in a scintillation counter.

Experiment 2 duplicated the conditions of Experiment 1 except for the substitution of 125 femtomoles of each of 5' fluorescein-labelled oligonucleotides J and D in the place of the 250 femtomoles of 5' fluorescein-labelled oligonucleotide B.

In Experiment 3, the conditions of Experiment 1 were duplicated except for the substitution of 83 femtomoles of each of the 5' fluorescein-labelled oligonucleotides J, G, and D for the 250 femtomoles of 5' fluorescein-labelled oligonucleotide B of Experiment 1.

In Experiment 4, the conditions of Experiment 1 were duplicated except for the substitution of 28 femtomoles of each of the 5' fluorescein-labelled oligonucleotides B, C, D, E, F, G, H, I, and J for the 250 femtomoles of 5' fluorescein-labelled oligonucleotide B of Experiment 1.

The results of these four experiments are summarized in Table V, wherein the percentage of sandwich hybridization complex captured by the bead is expressed as the ratio of ³²P oligonucleotide A captured per total amount of target present.

**TABLE V**

| Percentage of Experiment | Complex Captured |
|---|---|
| 1 | 5.4 |
| 2 | 14.2 |
| 3 | 21.4 |
| 4 | 61.2 |

As indicated in Table V, a roughly linear increase in hybridization efficiency was observed with increasing the number of different probes used. A 60% capture efficiency was achieved when all nine fluorescein oligonucleotides were used.

In general, the greater the number of points of stringency within a system, the less likely becomes the detection of false positives. In a conventional hybridization sandwich assay, the use of a separate probe for each of labelling and immobilization provides an additional point of stringency over use of a single probe for both purposes in that detection of a target sequence requires the occurrence of two independent events, i.e., the hybridization of both probes to the target. Consequently, it is believed that by the use of several first probes, the points of stringency are multiplied linearly, so that the efficiency of detection of a particular target sequence is increased relatively to the efficiency of detection of an incorrect sequence. Similarly, the use of a non-hybridization reaction to attach the first probe to the support serves to minimize detection of false positives by introducing a point of stringency associated with an antibody/ antigen interaction into a system which already has a nucleic acid-related point of stringency and which would otherwise have only another nucleic acid- related point of stringency in its place.

In the following example, the linearity of the capture efficiency of a hybridization complex over a range of target concentrations (10 femtomoles to 16 attomoles) was investigated in two sets of experiments.

### Example 3

In order to determine the effect of target concentration on the efficiency and sensitivity of the immuno-hybridization assay according to the present invention when run in the presence of extraneous DNA, the concentration of a target was varied from 10 femtomoles to 16 attomoles.

In each of six hybridization reactions, a solution of 111 femtomoles of each of 5' fluorescein-labelled oligonucleotides B, C, D, E, F, G, H, I, and J; 10µg of human placental DNA (available from Sigma Chemical Company, St. Louis, Missouri); and 100 femtomoles of ³²P-labelled oligonucleotide A in 5 X SSPE were prepared. To this basic solution, a varying amount of Φ2 SS target was added. In Experiment 1, 10 femtomoles of target were added. In Experiment 2, 2 femtomoles of target was added. In Experiments 3, 4 and 5, 0.4 femtomoles, 0.08 femtomoles, and 0.016 femtomoles, respectively, of Φ2SS target were added to the basic solution. In the Control experiment, no target was added.

The samples were boiled for 5 minutes and incubated for 1 hour at 50 ° C. Each sample was diluted with 400µl of 5 X SSPE containing 0.1% bovine gammaglobulin (Sigma Chemical Company, St. Louis, Missouri), and 0.1% sodium azide (Aldrich Chemical, Milwaukee, Wisconsin). One anti-fluorescein-coated bead was added to each solution, and each solution was mixed at 220 rpm for 3 hours at 25 ° C. Each bead was then washed sequentially in 1ml of 5 X SSPE for 5 minutes at 25°C and in 1 ml of 5 X SSPE for 5 minutes at 37 °C. Each of the beads was then counted on a scintillation counter. In Table VI, the percentage of sandwich hybridization complexes captured by the bead was calculated to be (³²P-labelled oligonucleotide A captured by the experimental bead - ³²P-labelled oligonucleotide A captured by the control bead) / (total amount of target present in the experiment) and averaged for two runs of each experiment.

**TABLE VI**

| cpm Captured Experiment | % Complex by Bead | Captured |
|---|---|---|
| 1 | 13,182 | 53 (± 10) |
| 2 | 2,479 | 48 (± 9) |
| 3 | 630 | 52 (± 11) |
| 4 | 180 | 38 (± 13) |
| 5 | 143 | 53 (± 9) |
| Control | 122 | 0 |

As indicated by the results in Table VI, the immuno-hybridization assay according to the present invention may detect the presence of target DNA in the attomole range as efficiently as in the femtomole range. Thus, the capture efficiency of the sandwich hybridization complex does not appear to be dependent upon the concentration of the target. The sensitivity of this system appears to be limited only by the specific activity of the radioactively-labelled probe. Thus, an immuno-hybridization assay according to the present invention may be used to detect the presence of a very small quantity of DNA with very few manipulations in a short period of time (4 to 5 hours).

In order to increase the sensitivity of the immuno-hybridization assay according to the present invention, a series of experiments was performed wherein a ³²P-labelled nick-translated DNA probe replaced the ³²P-labelled oligonucleotide probe of the previous examples. As indicated in the following Example, the greater length of a nick-translated probe allows a larger amount of label to be attached, so that lower target concentrations may be detected.

It is expected that numerous-modifications and variations will occur to those skilled in the art upon consideration of the present invention. For example, the component elements necessary to test a sample for the presence of a particular target DNA may be assembled in advance in the form of a kit. Specifically, a first probe complementary to a selected target and bound to a first immunological agent, a second probe bound to a reporter group and complementary to a different portion of the target than the first probe, and a second immunological agent bound to a support may be included in such a kit as separately packaged components. Such a kit may be used to detect the presence of and to quantify the target for which it was designed by combining the probes and support with a sample to be tested for target prepared, for example, according to the procedure of Ranki, et al., Curr. Top. Microbiol. Immunol., 104: 317-318 (1983).

Similarly, a kit may be prepared by associated containers of a reporter-bound second probe, a second immunological agent-bound support and a mixture or separate container of several first probes, each being bound to a first immunological agent. Although the sequences of a plurality of first probes may overlap to some extent where required (i.e., different but overlapping), it is particularly desirable from the standpoint of assay sensitivity that the sequence of the second probe be separate (i.e., non-overlapping) from as well as different from that of any first probe in order to ensure that as much immobilized target as possible is labelled.

In addition, although the present invention has been described in terms of a system employing anti-fluorescein coated beads, materials are readily available for practicing the present invention with complexing agents. For example, agarose-bound lectins and biotinylated agarose are available from Vector Laboratories, Inc., Burlingame, California. Avidin-coated polymethacrylate spheres and biotin-labelled RNA may be obtained by the procedure of Manning, et al., Chromosoma (Berl.), 53: 107-117 (1979).

Therefore, it is intended that the present invention include all such equivalent variations which come within the scope of the invention as claimed.

## Claims (Claims for the following Contracting State(s): BE, CH, DE, FR, GB, IT, LI, LU, NL, SE)

1. A method for the isolation of a selected target nucleic acid sequence from a solution, said method involving the formation of a sandwich complex of:
(a) a first single-stranded nucleic acid probe hybridized to a complementary first portion of the target, said first probe being attached to a first complexing agent for immobilizing said probe to a solid support bearing a second complexing agent capable of stably binding to the first complexing agent; (b) the target nucleic acid sequence; and (c) a second single-stranded nucleic acid probe hybridized to a complementary second portion of the target, said second probe being attached to a reporter group; **characterized in that**: said sandwich complex is formed in solution prior to immobilization of the first probe to the solid support; **in that** said first and second complexing agents are selected from the group of specific binding pairs consisting of an antigen and an antibody, and a lectin and a carbohydrate and **in that** said first complexing agent is attached to said first single-stranded nucleic acid probe at the 5' end thereof, and wherein the specific binding pairs do not consist of sulphonated DNA as the antigen and anti-sulphone monoclonal antibodies as the antibody.

2. The method as recited in Claim 1 further **characterized by** the step of: separating the solution from the immobilized hybrid sequence.

3. The method as recited in Claim 1 further **characterized in that** the target sequence is a double-stranded sequence and the method as recited in Claim 1 further **characterized by** the step of making a single-stranded portion of a double-stranded sequence available for solution hybridization.

4. The method as recited in claim 1 wherein the target sequence is a single-stranded sequence.

5. The method as recited in claim 1 wherein the first complexing agent is an antibody and wherein the second complexing agent is an antibody.

6. The method as recited in claim 1 wherein the first complexing agent is an antigen and wherein the second complexing agent is an antibody.

7. The method as recited in claim 5 or 6 wherein the antigen is fluorescein and wherein the antibody is an anti-fluorescein antibody.

8. The method as recited in claim 1 wherein the first complexing agent is a carbohydrate and wherein the second complexing agent is a lectin.

9. The method as recited in claim 1 wherein the first complexing agent is a lectin and wherein the second complexing agent is a carbohydrate.

10. The method as recited in claim 1 further comprising the step of: (d) assaying for the reporter group.

11. The method as recited in claim 10 wherein the target sequence is a double-stranded sequence and wherein the method as recited in claim 10 further comprises the step of making a single-stranded portion of a double-stranded sequence available for hybridization.

12. The method as recited in claim 10 wherein the target sequence is a single-stranded sequence.

13. The method an [sic] recited in claim 10 wherein the first complexing agent is an antibody and wherein the second complexing agent is an antigen.

14. The method as recited in claim 10 wherein the first complexing agent, [sic] is an antigen and wherein the second complexing agent is an antibody.

15. The method as recited in claim 13 or 14 wherein the antigen is fluorescein and wherein the antibody is an anti-fluorescein antibody.

16. The method as recited in claim 10 wherein the first complexing agent is a carbohydrate and wherein the second complexing agent is a lectin.

17. The method as recited in claim 10 wherein the first complexing agent is a lectin and wherein the second complexing agent is a carbohydrate.

18. The method as recited in claim 1 wherein the reporter group comprises an isotopic label covalently attached to the second probe sequence.

19. The method as recited in claim 1 wherein the reporter group comprises a radioactive heavy metal attached to the second probe by a chelating moiety.

20. The method as recited in claim 1 wherein the reporter group is non-radioactive.

21. The method as recited in claim 20 wherein the non-radioactive reporter group comprises biotin and wherein the method according to claim 1 further comprises the steps of:
complexing the biotin in the reporter group with an avidin moiety;
forming a complex of the avidin with a biotinylated calf alkaline phosphatase moiety;
and
reacting the avidin-complexed biotinylated calf alkaline phosphatase moiety with methylumberliferone [sic] phosphate.

22. The method as recited in Claim 1, comprising the steps of:
exposing the target nucleic acid sequence to at least two first probes, each having a nucleic acid sequence complementary to a different portion of the target nucleic acid sequence and each having a support-binding portion;
hybridizing in solution the target nucleic acid sequence with at least one of the first probes;
attaching the support-binding portion of at least one of the first probes to a first probe-binding protein on a solid support; and
introducing a second, single-stranded, nucleic acid probe having a sequence complementary to a portion of the target nucleic acid sequence which is separate from any portion complementary to any first probe, and being attached to a reporter group.

## Claims (Claims for the following Contracting State(s): AT)

1. A method for the isolation of a selected target nucleic acid sequence from a solution, said method involving the formation of a sandwich complex of:
(a) a first single-stranded nucleic acid probe hybridized to a complementary first portion of the target, said first probe being attached to a first complexing agent for immobilizing said probe to a solid support bearing a second complexing agent capable of stably binding to the first complexing agent; (b) the target nucleic acid sequence; and (c) a second single-stranded nucleic acid probe hybridized to a complementary second portion of the target, said second probe being attached to a reporter group; **characterized in that**: said sandwich complex is formed in solution prior to immobilization of the first probe to the solid support; **in that** said first and second complexing agents are selected from the group of specific binding pairs consisting of an antigen and an antibody, and a lectin and a carbohydrate and **in that** said first complexing agent is attached to said first single-stranded nucleic acid probe at the 5' end thereof.

2. The method as recited in Claim 1 further **characterized by** the step of: separating the solution from the immobilized hybrid sequence.

3. The method as recited in Claim 1 further **characterized in that** the target sequence is a double-stranded sequence and the method as recited in Claim 1 further **characterized by** the step of making a single-stranded portion of a double-stranded sequence available for solution hybridization.

4. The method as recited in claim 1 wherein the target sequence is a single-stranded sequence.

5. The method as recited in claim 1 wherein the first complexing agent is an antibody and wherein the second complexing agent is an antibody.

6. The method as recited in claim 1 wherein the first complexing agent is an antigen and wherein the second complexing agent is an antibody.

7. The method as recited in claim 5 or 6 wherein the antigen is fluorescein and wherein the antibody is an anti-fluorescein antibody.

8. The method as recited in claim 1 wherein the first complexing agent is a carbohydrate and wherein the second complexing agent is a lectin.

9. The method as recited in claim 1 wherein the first complexing agent is a lectin and wherein the second complexing agent is a carbohydrate.

10. The method as recited in claim 1 further comprising the step of: (d) assaying for the reporter group.

11. The method as recited in claim 10 wherein the target sequence is a double-stranded sequence and wherein the method as recited in claim 10 further comprises the step of making a single-stranded portion of a double-stranded sequence available for hybridization.

12. The method as recited in claim 10 wherein the target sequence is a single-stranded sequence.

13. The method an [sic] recited in claim 10 wherein the first complexing agent is an antibody and wherein the second complexing agent is an antigen.

14. The method as recited in claim 10 wherein the first complexing agent, [sic] is an antigen and wherein the second complexing agent is an antibody.

15. The method as recited in claim 13 or 14 wherein the antigen is fluorescein and wherein the antibody is an anti-fluorescein antibody.

16. The method as recited in claim 10 wherein the first complexing agent is a carbohydrate and wherein the second complexing agent is a lectin.

17. The method as recited in claim 10 wherein the first complexing agent is a lectin and wherein the second complexing agent is a carbohydrate.

18. The method as recited in claim 1 wherein the reporter group comprises an isotopic label covalently attached to the second probe sequence.

19. The method as recited in claim 1 wherein the reporter group comprises a radioactive heavy metal attached to the second probe by a chelating moiety.

20. The method as recited in claim 1 wherein the reporter group is non-radioactive.

21. The method as recited in claim 20 wherein the non-radioactive reporter group comprises biotin and wherein the method according to claim 1 further comprises the steps of:
complexing the biotin in the reporter group with an avidin moiety;
forming a complex of the avidin with a biotinylated calf alkaline phosphatase moiety;
and
reacting the avidin-complexed biotinylated calf alkaline phosphatase moiety with methylumberliferone [sic] phosphate.

22. The method as recited in Claim 1, comprising the steps of:
exposing the target nucleic acid sequence to at least two first probes, each having a nucleic acid sequence complementary to a different portion of the target nucleic acid sequence and each having a support-binding portion;
hybridizing in solution the target nucleic acid sequence with at least one of the first probes;
attaching the support-binding portion of at least one of the first probes to a first probe-binding protein on a solid support; and
introducing a second, single-stranded, nucleic acid probe having a sequence complementary to a portion of the target nucleic acid sequence which is separate from any portion complementary to any first probe, and being attached to a reporter group.

## Patentansprüche (Patentansprüche für folgende(n) Vertragsstaat(en): BE, CH, DE, FR, GB, IT, LI, LU, NL, SE)

1. Verfahren zur Isolierung einer ausgewählten Target-Nukleinsäuresequenz aus einer Lösung, wobei das Verfahren das Bilden eines Sandwich-Komplexes aus:
(a) einer ersten einzelsträngigen Nukleinsäure-Sonde, die an einen komplementären ersten Abschnitt des Targets hybridisiert ist, wobei die erste Sonde an einen ersten Komplexbildner zum Immobilisieren der Sonde an einem festen Träger geheftet ist, welcher einen zweiten Komplexbildner trägt, der in der Lage ist, sich stabil an den ersten Komplexbildner zu binden;
(b) der Target-Nukleinsäuresequenz; und
(c) einer zweiten einzelsträngigen Nukleinsäure-Sonde, die an einen komplementären zweiten Abschnitt des Targets hybridisiert ist, wobei die zweite Sonde an eine Reportergruppe angeheftet ist;
einschließt, **dadurch gekennzeichnet, daß**: der Sandwich-Komplex in Lösung vor dem Immobilisieren der ersten Sonde an dem festen Träger gebildet wird und daß der erste und zweite Komplexbildner aus der Gruppe spezifischer Bindungspaare, bestehend aus einem Antigen und einem Antikörper sowie einem Lektin und einem Kohlehydrat ausgewählt sind und dadurch, daß der erste Komplexbildner an die erste einzelsträngige Nukleinsäure-Sonde an deren 5'-Ende geheftet ist und wobei die spezifischen Bindungspaare nicht aus sulfonierter DNA als dem Antigen und Anti-Sulfonmonoklonalen Antikörpern als dem Antikörper bestehen.

2. Verfahren nach Anspruch 1, weiter **gekennzeichnet durch** den Schritt des: Abtrennens der Lösung von der immobilisierten Hybridsequenz.

3. Verfahren nach Anspruch 1, weiter **dadurch gekennzeichnet, daß** die Target-Sequenz eine doppelsträngige Sequenz ist und das Verfahren nach Anspruch 1 weiter durch den Schritt des Herstellens eines einzelsträngigen Abschnittes einer doppelsträngigen Sequenz, die für die Lösungshybridisierung zur Verfügung steht, gekennnzeichnet ist.

4. Verfahren nach Anspruch 1, bei dem die Target-Sequenz eine einzelsträngige Sequenz ist.

5. Verfahren nach Anspruch 1, bei dem der erste Komplexbildner ein Antikörper ist und bei dem der zweite Komplexbildner ein Antikörper ist.

6. Verfahren nach Anspruch 1, bei dem der erste Komplexbildner ein Antigen ist und bei dem der zweite Komplexbildner ein Antikörper ist.

7. Verfahren nach Anspruch 5 oder 6, bei dem das Antigen Fluorescein ist und bei dem der Antikörper ein Anti-Fluorescein-Antikörper ist.

8. Verfahren nach Anspruch 1, bei dem der erste Komplexbildner ein Kohlehydrat ist und bei dem der zweite Komplexbildner ein Lektin ist.

9. Verfahren nach Anspruch 1, bei dem der erste Komplexbildner ein Lektin ist und bei der zweite Komplexbildner ein Kohlehydrat ist.

10. Verfahren nach Anspruch 1, das weiterhin den Schritt aufweist: (d) Prüfen auf die Reportergruppe.

11. Verfahren nach Anspruch 10, bei dem die Target-Sequenz eine doppelsträngige Sequenz ist und bei dem das Verfahren nach Anspruch 10 weiter den Schritt des Herstellens eines einzelsträngigen Abschnittes einer doppelsträngigen Sequenz, die für die Hybridisierung zur Verfügung steht, aufweist

12. Verfahren nach Anspruch 10, bei dem die Target-Sequenz eine einzelsträngige Sequenz ist

13. Verfahren nach Anspruch 10, bei dem der erste Komplexbildner ein Antikörper ist und bei dem der zweite Komplexbildner ein Antigen ist

14. Verfahren nach Anspruch 10, bei dem der erste Komplexbildner ein Antigen ist und bei dem der zweite Komplexbildner ein Antikörper ist.

15. Verfahren nach Anspruch 13 oder 14, bei dem das Antigen Fluorescein ist und bei dem der Antikörper ein Anti-Fluorescein-Antikörper ist.

16. Verfahren nach Anspruch 10, bei dem der erste Komplexbildner ein Kohlehydrat ist und bei dem der zweite Komplexbildner ein Lektin ist.

17. Verfahren nach Anspruch 10, bei dem der erste Komplexbildner ein Lektin ist und bei dem der zweite Komplexbildner ein Kohlehydrat ist.

18. Verfahren nach Anspruch 1, bei dem die Reportergruppe eine Isotopen-Markierung aufweist, die kovalent an die zweite Sonden-Sequenz angeheftet ist.

19. Verfahren nach Anspruch 1, bei dem die Reportergruppe ein radioaktives Schwermetall aufweist, das durch einen chelatbildenden Rest an die zweite Sonde geheftet ist.

20. Verfahren nach Anspruch 1, bei dem die Reportergruppe nicht radioaktiv ist.

21. Verfahren nach Anspruch 20, bei dem die nicht radioaktive Reportergruppe Biotin aufweist und bei dem das Verfahren nach Anspruch 1 weiter die Schritte aufweist;
Komplexieren des Biotins in der Reportergruppe mit einem Avidinrest;
Bilden eines Komplexes des Avidins mit einem biotinylierten Rest alkalischer Kälber-Phosphatase; und
Umsetzen des mit dem Avidin komplexierten biotinylierten Rest alkalischer Kälber-Phosphatase mit Methylumberliferonphosphat.

22. Verfahren nach Anspruch 1, das die Schritte umfaßt:
Aussetzen der Target-Nukleinsäuresequenz gegenüber wenigstens zwei Sonden, von denen jede eine Nukleinsauresequenz komplementär zu einem unterschiedlichen Abschnitt der Target-Nukleinsäuresequenz hat und wobei jede einen trägerbindenden Abschnitt hat;
Hybridisieren der Target-Nukleinsäuresequenz mit wenigstes einer der ersten Sonden in Lösung;
Anheften des trägerbindenden Abschnittes wenigstens einer der ersten Sonden an ein erstes sondenbindendes Protein auf einem festen Träger; und
Einführen einer zweiten, einzelsträngigen Nukleinsäure-Sonde, die eine Sequenz komplementär zu einem Abschnitt der Target-Nukleinsäure-Sequenz aufweist, der von irgendeinem Abschnitt, der komplementär zu irgendeiner ersten Sonde ist, getrennt ist und die an eine Reportergruppe angeheftet ist.

## Patentansprüche (Patentansprüche für folgende(n) Vertragsstaat(en): AT)

1. Verfahren zur Isolierung einer ausgewählten Target-Nukleinsäuresequenz aus einer Lösung, wobei das Verfahren das Bilden eines Sandwich-Komplexes aus:
(a) einer ersten einzelsträngigen Nukleinsäure-Sonde, die an einen komplementären ersten Abschnitt des Targets hybridisiert ist, wobei die erste Sonde an einen ersten Komplexbildner zum Immobilisieren der Sonde an einem festen Träger geheftet ist, welcher einen zweiten Komplexbildner trägt, der in der Lage ist, sich stabil an den ersten Komplexbildner zu binden;
(b) der Target-Nukleinsäuresequenz; und
(c) einer zweiten einzelsträngigen Nukleinsäure-Sonde, die an einen komplementären zweiten Abschnitt des Targets hybridisiert ist, wobei die zweite Sonde an eine Reportergruppe angeheftet ist;
einschließt, **dadurch gekennzeichnet, daß**: der Sandwich-Komplex in Lösung vor dem Immobilisieren der ersten Sonde an dem festen Träger gebildet wird, daß der erste und zweite Komplexbildner aus der Gruppe spezifischer Bindungspaare, bestehend aus einem Antigen und einem Antikörper, sowie einem Lektin und einem Kohlehydrat ausgewählt sind und dadurch, daß der erste Komplexbildner an die erste einzelsträngige Nukleinsäure an deren 5'-Ende geheftet ist.

2. Verfahren nach Anspruch 1, weiter **gekennzeichnet durch** den Schritt des: Abtrennens der Lösung von der immobilisierten Hybridsequenz.

3. Verfahren nach Anspruch 1, weiter **dadurch gekennzeichnet, daß** die Target-Sequenz eine doppelsträngige Sequenz ist und das Verfahren nach Anspruch 1 weiter durch den Schritt des Herstellens eines einzelsträngigen Abschnittes einer doppelsträngigen Sequenz, die für die Lösungshybridisierung zur Verfügung steht, gekennnzeichnet ist.

4. Verfahren nach Anspruch 1, bei dem die Target-Sequenz eine einzelsträngige Sequenz ist.

5. Verfahren nach Anspruch 1, bei dem der erste Komplexbildner ein Antikörper ist und bei dem der zweite Komplexbildner ein Antikörper ist.

6. Verfahren nach Anspruch 1, bei dem der erste Komplexbildner ein Antigen ist und bei dem der zweite Komplexbildner ein Antikörper ist.

7. Verfahren nach Anspruch 5 oder 6, bei dem das Antigen Fluorescein ist und bei dem der Antikörper ein Anti-Fluorescein-Antikörper ist.

8. Verfahren nach Anspruch 1, bei dem der erste Komplexbildner ein Kohlehydrat ist und bei dem der zweite Komplexbildner ein Lektin ist.

9. Verfahren nach Anspruch 1, bei dem der erste Komplexbildner ein Lektin ist und bei der zweite Komplexbildner ein Kohlehydrat ist.

10. Verfahren nach Anspruch 1, das weiterhin den Schritt aufweist: (d) Prüfen auf die Reportergruppe.

11. Verfahren nach Anspruch 10, bei dem die Target-Sequenz eine doppelsträngige Sequenz ist und bei dem das Verfahren nach Anspruch 10 weiter den Schritt des Herstellens eines einzelsträngigen Abschnittes einer doppelsträngigen Sequenz, die für die Hybridisierung zur Verfügung steht, aufweist

12. Verfahren nach Anspruch 10, bei dem die Target-Sequenz eine einzelsträngige Sequenz ist

13. Verfahren nach Anspruch 10, bei dem der erste Komplexbildner ein Antikörper ist und bei dem der zweite Komplexbildner ein Antigen ist

14. Verfahren nach Anspruch 10, bei dem der erste Komplexbildner ein Antigen ist und bei dem der zweite Komplexbildner ein Antikörper ist.

15. Verfahren nach Anspruch 13 oder 14, bei dem das Antigen Fluorescein ist und bei dem der Antikörper ein Anti-Fluorescein-Antikörper ist.

16. Verfahren nach Anspruch 10, bei dem der erste Komplexbildner ein Kohlehydrat ist und bei dem der zweite Komplexbildner ein Lektin ist.

17. Verfahren nach Anspruch 10, bei dem der erste Komplexbildner ein Lektin ist und bei dem der zweite Komplexbildner ein Kohlehydrat ist.

18. Verfahren nach Anspruch 1, bei dem die Reportergruppe eine Isotopen-Markierung aufweist, die kovalent an die zweite Sonden-Sequenz angeheftet ist.

19. Verfahren nach Anspruch 1, bei dem die Reportergruppe ein radioaktives Schwermetall aufweist, das durch einen chelatbildenden Rest an die zweite Sonde geheftet ist.

20. Verfahren nach Anspruch 1, bei dem die Reportergruppe nicht radioaktiv ist.

21. Verfahren nach Anspruch 20, bei dem die nicht radioaktive Reportergruppe Biotin aufweist und bei dem das Verfahren nach Anspruch 1 weiter die Schritte aufweist;
Komplexieren des Biotins in der Reportergruppe mit einem Avidinrest;
Bilden eines Komplexes des Avidins mit einem biotinylierten Rest alkalischer Kälber-Phosphatase; und
Umsetzen des mit dem Avidin komplexierten biotinylierten Rest alkalischer Kälber-Phosphatase mit Methylumberliferonphosphat.

22. Verfahren nach Anspruch 1, das die Schritte umfaßt:
Aussetzen der Target-Nukleinsäuresequenz gegenüber wenigstens zwei Sonden, von denen jede eine Nukleinsauresequenz komplementär zu einem unterschiedlichen Abschnitt der Target-Nukleinsäuresequenz hat und wobei jede einen trägerbindenden Abschnitt hat;
Hybridisieren der Target-Nukleinsäuresequenz mit wenigstes einer der ersten Sonden in Lösung;
Anheften des trägerbindenden Abschnittes wenigstens einer der ersten Sonden an ein erstes sondenbindendes Protein auf einem festen Träger; und
Einführen einer zweiten, einzelsträngigen Nukleinsäure-Sonde, die eine Sequenz komplementär zu einem Abschnitt der Target-Nukleinsäure-Sequenz aufweist, der von irgendeinem Abschnitt, der komplementär zu irgendeiner ersten Sonde ist, getrennt ist und die an eine Reportergruppe angeheftet ist.

## Revendications (Revendications pour l'(les) Etat(s) contractant(s) suivant(s): AT, BE, CH, DE, FR, GB, IT, LI, LU, NL, SE)

1. Procédé pour l'isolation d'une séquence d'acides nucléiques cible choisie à partir d'une solution, ledit procédé impliquant la formation d'un complexe sandwich de :
(a) une première sonde d'acides nucléiques simple brin, hybridée à une première partie complémentaire de la cible, ladite première sonde étant fixée à un premier agent complexant pour immobiliser ladite sonde sur un support solide portant un second agent complexant capable de se lier de façon stable au premier agent complexant ; (b) la séquence d'acides nucléiques cible ; et (c) une seconde sonde d'acides nucléiques simple brin, hybridée à une seconde partie complémentaire de la cible, ladite seconde sonde étant fixée à un groupe rapporteur ;
**caractérisé en ce que** ledit complexe sandwich est formé en solution avant l'immobilisation de la première sonde au support solide ; **en ce que** lesdits premier et second agents complexants sont choisis dans le groupe de paires spécifiques de liaison constituées d'un antigène et d'un anticorps, et d'une lectine et d'un carbohydrate, et **en ce que** ledit premier agent complexant est fixé à ladite première sonde d'acides nucléiques simple brin au niveau de son extrémité 5', et dans lequel les paires spécifiques de liaison ne sont pas constituées d'ADN sulfoné comme l'antigène, et d'anticorps monoclonaux anti-sulfone comme l'anticorps.

2. Procédé selon la revendication 1,
caractérisé de plus par l'étape consistant à séparer la solution de la séquence hybride immobilisée.

3. Procédé selon la revendication 1,
caractérisé de plus en ce que la séquence cible est une séquence double brin, et procédé selon la revendication 1 caractérisé de plus par l'étape consistant à rendre disponible une partie simple brin d'une séquence double brin pour l'hybridation en solution.

4. Procédé selon la revendication 1, dans lequel la séquence cible est une séquence simple brin.

5. Procédé selon la revendication 1, dans lequel le premier agent complexant est un anticorps et dans lequel le second agent complexant est un anticorps.

6. Procédé selon la revendication 1, dans lequel le premier agent complexant est un antigène et dans lequel le second agent complexant est un anticorps.

7. Procédé selon la revendication 5 ou 6, dans lequel l'antigène est de la fluorescéine, et dans lequel l'anticorps est un anticorps anti-fluorescéine.

8. Procédé selon la revendication 1, dans lequel le premier agent complexant est un carbohydrate et dans lequel le second agent complexant est une lectine.

9. Procédé selon la revendication 1, dans lequel le premier agent complexant est une lectine et dans lequel le second agent complexant est un carbohydrate.

10. Procédé selon la revendication 1, comprenant de plus l'étape consistant à (d) tester le groupe rapporteur.

11. Procédé selon la revendication 10, dans lequel la séquence cible est une séquence double brin et dans lequel le procédé selon la revendication 10 comprend de plus l'étape consistant à rendre une partie simple brin d'une séquence double brin disponible pour l'hybridation.

12. Procédé selon la revendication 10, dans lequel la séquence ci-ble est une séquence simple brin.

13. Procédé selon [sic] la revendication 10, dans lequel le premier agent complexant est un anticorps et dans lequel le second agent complexant est un antigène.

14. Procédé selon la revendication 10, dans lequel le premier agent complexant [sic] est un antigène et dans lequel le second agent complexant est un anticorps.

15. Procédé selon la revendication 13 ou 14, dans lequel l'antigène est de la fluorescéine, et dans lequel l'anticorps est un anticorps anti-fluorescéine.

16. Procédé selon la revendication 10, dans lequel le premier agent complexant est un carbohydrate et dans lequel le second agent complexant est une lectine.

17. Procédé selon la revendication 10, dans lequel le premier agent complexant est une lectine et dans lequel le second agent complexant est un carbohydrate.

18. Procédé selon la revendication 1, dans lequel le groupe rapporteur comprend un marqueur isotopique fixé de façon covalente à la seconde séquence sonde.

19. Procédé selon la revendication 1, dans lequel le groupe rapporteur comprend un métal lourd radioactif fixé à la seconde sonde par une fraction chélatante.

20. Procédé selon la revendication 1, dans lequel le groupe rapporteur est non-radioactif.

21. Procédé selon la revendication 20, dans lequel le groupe rapporteur non-radioactif comprend de la biotine et dans lequel le procédé selon la revendication 1 comprend de plus les étapes consistant à :
- complexer la biotine dans le groupe rapporteur avec une fraction d'avidine ;
- former un complexe de l'avidine avec une fraction de phosphatase alcaline de veau biotinylée ; et
- faire réagir la fraction de phosphatase alcaline de veau biotinylée complexée par l'avidine avec du phosphate [sic] de méthylumbelliférone.

22. Procédé selon la revendication 1, comprenant les étapes consistant à :
- exposer la séquence d'acides nucléiques cible à au moins deux premières sondes, chacune ayant une séquence d'acides nucléiques complémentaire d'une partie différente de la séquence d'acides nucléiques cible et chacune ayant une partie de liaison de support ;
- hybrider en solution la séquence d'acides nucléiques cible avec au moins une des premières sondes ;
- fixer la partie de liaison de support d'au moins une des premières sondes à une première protéine de liaison de sonde sur un support solide ; et
- introduire une seconde sonde d'acides nucléiques double brin ayant une séquence complémentaire d'une partie de la séquence d'acides nucléiques cible qui est séparée d'une partie quelconque complémentaire d'une première sonde quelconque, et étant fixée à un groupe rapporteur.

## Revendications (Revendications pour l'(les) Etat(s) contractant(s) suivant(s): AT)

1. Procédé pour l'isolation d'une séquence d'acides nucléiques cible choisie à partir d'une solution, ledit procédé impliquant la formation d'un complexe sandwich de :
(a) une première sonde d'acides nucléiques simple brin, hybridée à une première partie complémentaire de la cible, ladite première sonde étant fixée à un premier agent complexant pour immobiliser ladite sonde sur un support solide portant un second agent complexant capable de se lier de façon stable au premier agent complexant ; (b) la séquence d'acides nucléiques cible ; et
(c) une seconde sonde d'acides nucléiques simple brin, hybridée à une seconde partie complémentaire de la cible, ladite seconde sonde étant fixée à un groupe rapporteur ;
**caractérisé en ce que** ledit complexe sandwich est formé en solution avant l'immobilisation de la première sonde au support solide ; **en ce que** lesdits premier et second agents complexants sont choisis dans le groupe de paires spécifiques de liaison constituées d'un antigène et d'un anticorps, et d'une lectine et d'un carbohydrate, et **en ce que** ledit premier agent complexant est fixé à ladite première sonde d'acides nucléiques simple brin au niveau de son extrémité 5'.

2. Procédé selon la revendication 1,
caractérisé de plus par l'étape consistant à séparer la solution de la séquence hybride immobilisée.

3. Procédé selon la revendication 1,
caractérisé de plus en ce que la séquence cible est une séquence double brin, et procédé selon la revendication 1 caractérisé de plus par l'étape consistant à rendre disponible une partie simple brin d'une séquence double brin pour l'hybridation en solution.

4. Procédé selon la revendication 1, dans lequel la séquence cible est une séquence simple brin.

5. Procédé selon la revendication 1, dans lequel le premier agent complexant est un anticorps et dans lequel le second agent complexant est un anticorps.

6. Procédé selon la revendication 1, dans lequel le premier agent complexant est un antigène et dans lequel le second agent complexant est un anticorps.

7. Procédé selon la revendication 5 ou 6, dans lequel l'antigène est de la fluorescéine, et dans lequel l'anticorps est un anticorps anti-fluorescéine.

8. Procédé selon la revendication 1, dans lequel le premier agent complexant est un carbohydrate et dans lequel le second agent complexant est une lectine.

9. Procédé selon la revendication 1, dans lequel le premier agent complexant est une lectine et dans lequel le second agent complexant est un carbohydrate.

10. Procédé selon la revendication 1, comprenant de plus l'étape consistant à (d) tester le groupe rapporteur.

11. Procédé selon la revendication 10, dans lequel la séquence cible est une séquence double brin et dans lequel le procédé selon la revendication 10 comprend de plus l'étape consistant à rendre une partie simple brin d'une séquence double brin disponible pour l'hybridation.

12. Procédé selon la revendication 10, dans lequel la séquence cible est une séquence simple brin.

13. Procédé selon [sic] la revendication 10, dans lequel le premier agent complexant est un anticorps et dans lequel le second agent complexant est un antigène.

14. Procédé selon la revendication 10, dans lequel le premier agent complexant [sic] est un antigène et dans lequel le second agent complexant est un anticorps.

15. Procédé selon la revendication 13 ou 14, dans lequel l'antigène est de la fluorescéine, et dans lequel l'anticorps est un anticorps anti-fluorescéine.

16. Procédé selon la revendication 10, dans lequel le premier agent complexant est un carbohydrate et dans lequel le second agent complexant est une lectine.

17. Procédé selon la revendication 10, dans lequel le premier agent complexant est une lectine et dans lequel le second agent complexant est un carbohydrate.

18. Procédé selon la revendication 1, dans lequel le groupe rapporteur comprend un marqueur isotopique fixé de façon covalente à la seconde séquence sonde.

19. Procédé selon la revendication 1, dans lequel le groupe rapporteur comprend un métal lourd radioactif fixé à la seconde sonde par une fraction chélatante.

20. Procédé selon la revendication 1, dans lequel le groupe rapporteur est non-radioactif.

21. Procédé selon la revendication 20, dans lequel le groupe rapporteur non-radioactif comprend de la biotine et dans lequel le procédé selon la revendication 1 comprend de plus les étapes consistant à :
- complexer la biotine dans le groupe rapporteur avec une fraction d'avidine ;
- former un complexe de l'avidine avec une fraction de phosphatase alcaline de veau biotinylée ; et
- faire réagir la fraction de phosphatase alcaline de veau biotinylée complexée par l'avidine avec du phosphate [sic] de méthylumbelliférone.

22. Procédé selon la revendication 1, comprenant les étapes consistant à :
- exposer la séquence d'acides nucléiques cible à au moins deux premières sondes, chacune ayant une séquence d'acides nucléiques complémentaire d'une partie différente de la séquence d'acides nucléiques cible et chacune ayant une partie de liaison de support ;
- hybrider en solution la séquence d'acides nucléiques cible avec au moins une des premières sondes ;
- fixer la partie de liaison de support d'au moins une des premières sondes à une première protéine de liaison de sonde sur un support solide ; et
- introduire une seconde sonde d'acides nucléiques double brin ayant une séquence complémentaire d'une partie de la séquence d'acides nucléiques cible qui est séparée d'une partie quelconque complémentaire d'une première sonde quelconque, et étant fixée à un groupe rapporteur.
